# EUROPÄISCHE PATENTSCHRIFT

(11) **EP 2 177 280 B1**
(45) Veröffentlichungstag und Bekanntmachung des Hinweises auf die Patenterteilung: **30.05.2012**
(21) Anmeldenummer: 09173008.5
(22) Anmeldetag: 14.10.2009
(51) Int. Cl.: B09B 3/00, C12M 1/107, D21B 1/36, D21C 1/02

(54) **Verfahren und Vorrichtung zur diskontinuierlichen Hydrolyse von organischen Substraten**
Method and device for discontinuous hydrolysis of organic substrates
Procédé et dispositif d'hydrolyse discontinue de substrats organiques

(30) Priorität: 16.10.2008 AT 16272008
(43) Veröffentlichungstag der Anmeldung: 21.04.2010
(73) Patentinhaber: BIOGAS SYSTEMS GMBH, 2320 SCHWECHAT (AT)
(72) Erfinder: Dauser, Hermann, 83395 Freilassing (DE)
(74) Vertreter: Babeluk, Michael

(56) Entgegenhaltungen:
- WO-A2-2008/011839
- US-A1- 2003 121 851
- US-A1- 2008 277 082

## Beschreibung

Die Erfindung betrifft ein Verfahren und eine Vorrichtung zur diskontinuierlichen Hydrolyse von organischen Substraten, insbesondere von Energiepflanzen, sowie pflanzlichen und tierischen Abfällen, mit einem Vorkonditionierungstank zur Aufnahme fester und/oder flüssiger organischer Substanzen, mit einer Beschickungseinrichtung zur Befüllung eines Hydrolizers mit den Substraten aus dem Vorkonditionierungstank, sowie mit einer Entspannungseinrichtung, die zur Druckentspannung der Substrate nach der Behandlung im Hydrolizer einen Expandertank aufweist.

Derartige Vorrichtungen und Verfahren dienen zur Vorbehandlung organischer Substrate, die nach der Aufbereitung in einem Hydrolizer (Einrichtung zur Thermodruckhydrolyse) einem Fermenter, beispielsweise einer Biogas- oder Biofuel-Anlage zugeführt werden.

Bei der Thermodruckhydrolyse kommt die Technologie der sogenannten "Steam-Explosion" zur Anwendung, welche beispielsweise aus Biogas- oder Biofuel-Anlagen bekannt ist. Die "Steam-Explosion" ist ein technischer Prozess, bei dem das Ausgangsmaterial auf bis zu 300°C, vorzugsweise 150°C bis 200°C aufgeheizt und unter ca. 3 bar bis 20 bar Überdruck gesetzt wird. Nachdem dieser Druck/- Temperatur-Zustand über eine bestimmte Verweilzeit eingehalten wurde, wird das Substrat spontan auf etwa Atmosphärendruck entspannt. Durch diese Schlagentspannung kommt es zur vollständigen Aufspaltung der Zellsubstanz. Die gesamte organische Substanz steht in der Folge verflüssigt für die weitere Verarbeitung zur Verfügung.

Aus der ursprünglich eingesetzten inhomogenen Substratmischung (wie beispielsweise Energiepflanzen, urbaner Biomüll, Ernteabfälle und industrielle Schlachtabfälle, etc.) wird so ein homogener Brei mit folgenden Eigenschaften:
- die Zellulose wird freigelegt;
- das Lignin wird gelöst;
- die Hemizellulose wird aufgeschlossen;
- die Stärke wird verkleistert;
- Hefen, Schimmelpilze und andere störende Mikroorganismen werden abgetötet;
- das Substrat wird sterilisiert;
- die Faserstoffe werden destabilisiert.

Die "Steam-Explosion" übernimmt somit - vor einer weiteren Substratverarbeitung, z.B. in einer Biogasanlage - weitgehend die Verfahrensschritte Hydrolyse und Homogenisierung, so dass beispielsweise im Fermentationsreaktor nur mehr eine freie, reine Methangärung abläuft.

Das Resultat dieser Vorbehandlung ist eine erhöhte Substratausbeute mit verbesserter Produktqualität, im Falle einer Biogasanlage z.B. eine erhöhte Substratabbaurate mit gesteigerter Gasproduktion und verbesserter Gasqualität. Typischer Weise steigt beispielsweise der spezifische Methangehalt (CH₄) an, während der schädliche Gehalt an Schwefelwasserstoff (H₂S) reduziert wird.

Die US 2003/0121851 A1 beschreibt ein Verfahren und eine Vorrichtung für die Behandlung biologisch abbaubarer organischer Abfälle. Bevor der organische Abfall der Thermodruckhydrolyse zugeführt wird, wird dem Substrat eine Lauge (KOH) zugeführt und das Substrat im Hydrolizer auf Temperaturen von 170°C bis 225°C und korrelierendem Dampfdruck unterworfen. Danach erfolgt eine Fest/- Flüssig-Separation. Das Substrat kann vor der Behandlung durch eine Dampfrückführung aus dem Hydrolizer vorgeheizt werden.

Ein sehr ähnliches Verfahren beschreibt die US 2008/0223793 A1, welche in Ergänzung zur oben zitierten US 2003/121851 ein Verfahren beschreibt, bei welchem die Abdampfbrüden kondensiert werden und zumindest zum Teil dem Rohsubstrat zugemischt werden. Durch die im Kondensat enthaltenen organischen Säuren soll eine Verbesserung des Hydrolyse-Grades erzielt werden.

Aus der WO 2008/011839 A2 ist beispielsweise eine Vorrichtung für die kontinuierliche und diskontinuierliche Hydrolyse organischer Substrate bekannt geworden. Die Anlage besteht im Wesentlichen aus einer Zerkleinerungseinrichtung für das inhomogene organische Substrat, aus welcher das Substrat in einen Zumessbehälter für den Hydrolizer gelangt. Nach der Behandlung des Substrates im Hydrolizer wird dieses in einer sogenannten "Overshooting Pipe" in einen Expandertank transportiert, aus welchem eine Abgasleitung zu einem Kondensator und eine Substratleitung zu einem Fermenter wegführen. Die Abgase werden einem Dampfkondensator zugeführt, der eine Wasserkühlung aufweist, wobei das dabei gewonnene Kondensat wieder in den Expandertank zurückgeführt wird. In der Substratleitung zum Fermenter ist ein Wärmetauscher angeordnet, dessen Abwärme über einen externen Wärmetauscherkreislauf zu einem als Vorheizeinrichtung ausgeführten Wärmetauscher führt, mit welchem das zugeführte Substrat im Bereich nach der Zerkleinerungseinrichtung erwärmt wird.

Gemäß einer weiteren Ausführungsvariante der WO 2008/011839 A2, die im kontinuierlichen Betrieb arbeitet, gelangt das organische Substrat nach der Zerkleinerungseinrichtung in einen Ausgleichstank und wird aus diesem mit einer Hochdruckpumpe kontinuierlich dem Hydrolizer zugeführt. Auch bei dieser Ausführungsvariante weist der Expandertank eine Abgasleitung auf, mit welcher der Heißdampf aus dem Expandertank einem Dampfkondensator mit externer Wasserkühlung zugeführt wird. Das entstehende Kondensat wird in den Expandertank rückgeführt.

Nachteilig bei den bekannten Einrichtungen und Verfahren ist, dass diese energetisch nicht optimiert sind und auch relativ komplex aufgebaut sind.

Aufgabe der Erfindung ist es, eine Vorrichtung zur diskontinuierlichen Hydrolyse von organischen Substraten betriebstechnisch und energietechnisch zu optimieren, wobei die Anlage kompakt dimensioniert sein soll.

Diese Aufgabe wird erfindungsgemäß dadurch gelöst, dass die Entspannungseinrichtung einen Dampfabscheider aufweist, der dem Expandertank vorgeschaltet und stromabwärts einer Druckblende angeordnet ist, wobei eine Einrichtung zur direkten Beaufschlagung der zugeführten organischen Substrate mit aus dem Dampfabscheider abgeschiedenem Heißdampf vorgesehen ist.

Aus den in der Thermodruckhydrolyse behandelten Substraten wird unmittelbar nach der Ausschleusung einer Teilmenge des Substrates durch Druckentspannung Heißdampf abgeschieden und zur Erhitzung der dem Hydrolyseverfahren zugeführten organischen Substraten eingesetzt. Der Heißdampf kann bevorzugt direkt in das angemaischte organische Substrat eingeblasen werden.

In einer bevorzugten Ausführungsvariante der Erfindung ist der Dampfabscheider als Zyklon ausgebildet und weist eine Dampfverteilereinheit, beispielsweise eine Düsenstockeinheit auf, die direkt im Vorkonditionierungstank für die organischen Substrate angeordnet ist.

Im Sinne einer weiteren Verbesserung des Energiemanagements der erfindungsgemäßen Vorrichtung weist die Beschickungseinrichtung zur Befüllung des Hydrolizers einen Druckbehälter auf, der neben einer ventilgesteuerten Beschickungsleitung bzw. Schleuse zum Hydrolizer eine weitere ventilgesteuerte Verbindungsleitung zur Herstellung eines Temperatur- und Druckausgleichs mit dem Hydrolizer aufweist. Wie weiter unten ausführlich beschrieben, können durch diese Einrichtung in rascher Abfolge Beschickungs- und Entnahmezyklen realisiert werden, bei welchen nur Teile des Hydrolizer-Volumens ausgetauscht werden, so dass ein quasikontinuierlicher Betrieb der Vorrichtung erreicht werden kann.

Gemäß einer vorteilhaften Ausführungsvariante der Erfindung kann der Druckbehälter der Beschickungseinrichtung mit einer Siebeinheit ausgestattet sein, welche zur Entwässerung des aus dem Vorkonditionierungstank zugeführten Substrates dient.

Weiters ist es insbesondere bei relativ trockenem, pflanzlichem Substrat von Vorteil, wenn die Beschickungseinrichtung eine in den Vorkonditionierungstank ragende Transportschnecke zur Förderung eines oberflächlichen Schwimmkoffers aufweist, der sich aus festen, faserigen Bestandteilen der vorzugsweise pflanzlichen Abfälle bildet. Dabei kann die Transportschnecke eine Siebeinheit aufweisen, die zur Vorentwässerung des geförderten Substrats dient.

Durch die oben genannten Maßnahmen werden die der Thermodruckhydrolyse zugeführten angemaischten, organischen Substrate zuvor entwässert, so dass nur noch geringere Substratmengen auf das für die Thermodruckhydrolyse erforderliche Temperaturniveau gebracht werden müssen.

Die Erfindung wird im Folgenden anhand von schematischen Darstellungen näher erläutert. Es zeigen:
- Fig. 1: eine erfindungsgemäße Vorrichtung zur diskontinuierlichen Hydro- lyse organischer Substrate; sowie
- Fig. 2: eine erfindungsgemäße Variante der Vorrichtung gemäß Fig. 1.

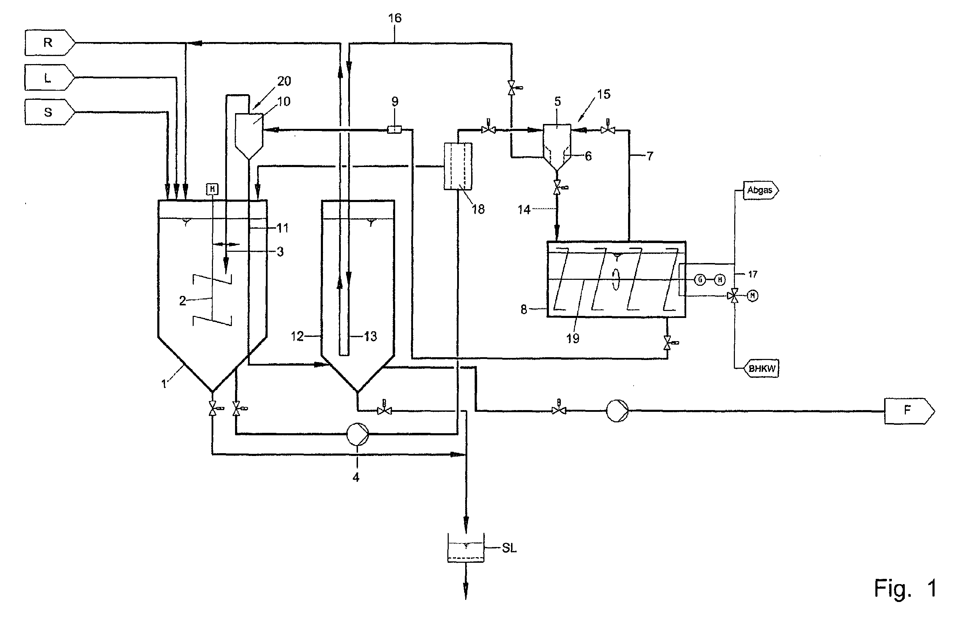

Die in Fig. 1 dargestellte Vorrichtung zur diskontinuierlichen Hydrolyse von organischen Substraten besteht im Wesentlichen aus folgenden Komponenten:
- einem Vorkonditionierungstank 1 zur Aufnahme flüssiger und fester organischer Substanzen mit einem Rührwerk 2 und einer Dampfverteilereinheit 3 (z.B. ausgebildet als Düsenstockeinheit);
- einer Transferpumpe 4 zur Entnahme der organischen Substrate aus dem Vorkonditionierungstank 1;
- einer Beschickungseinrichtung 15 mit einer Siebeinheit 6 zur Entwässerung des aus dem Vorkonditionierungstank 1 zugeführten Substrates mit einem Druckbehälter 5 (Blow Gun) samt Beschickungsleitung 14 und einer ventilgesteuerten Verbindungsleitung 7 zum Hydrolizer 8;
- einem Hydrolizer 8 zur Durchführung der Thermodruckhydrolyse;
- einer Entspannungseinrichtung 20 bestehend aus einer Druckblende 9, einem Dampfabscheider 10 (z.B. Zyklon) und einem Expanderrohr 11, welches in einen Expandertank 12 führt;
- einer Einrichtung 17 zur Abgasbeheizung des Hydrolizers 8;
- einem Expandertank 12 mit integriertem Wärmetauscher 13.

Mit der erfindungsgemäßen Vorrichtung wird festes Substrat S und/oder flüssiges Substrat L in hier nicht dargestellter Weise u.A. ggf. nach erforderlicher mechanischer Vorzerkleinerung mit einer Förderschnecke oder Pumpe aus bekannten Lagerbehältnissen (z.B. Bunker, Pufferbecken) in definierter Menge in den Vorkonditionierungstank 1 gefördert. Ein massives Rührwerk 2 mischt und homogenisiert die dickflüssige Masse bzw. das Wasser/Feststoff-Gemisch (Trockensubstanzgehalt üblicherweise >10% bis zu 50%, jedoch auch außerhalb dieses Bereiches möglich) und sorgt für die notwendige Homogenisierung. Bei zu trockenen Substraten wird Verdünnungswasser als Frischwasser, Brauchwasser oder Rezyklat R (z.B. aus den nachgeschalteten Prozessen) in definierter Menge zugegeben.

Ein trichterförmiger Boden des Vorkonditionierungstanks 1 mit Ablass ermöglicht die Separation von Schwerstoffen und Abführung in ein Sedimentlager SL

Im Vorkonditionierungstank 1 ist eine Dampfverteilereinheit 3, z.B. als Düsenstock, für die Zufuhr von Heißdampf integriert, die eine direkte Beheizung des Substrates durch Eindüsen des Abdampfes aus dem Dampfabscheider 10 ermöglicht. Damit erfolgt eine sehr effektive Form der Wärmerückgewinnung im Gegensatz zu bekannten Anlagen, bei welchen der Abdampf aus dem Expandertank in Kondensationsanlagen durch Abkühlung kondensiert wird.

Der Vorkonditionierungstank 1 ist für eine Aufenthaltszeit dimensioniert (ca. 1 bis 10 Stunden), die einen ausreichenden Wärmeübergang sowie eine Verkleisterung bzw. ein Aufquellen der Substrate ermöglicht. Diese Zeitspanne kann für unterschiedliche Substrate variiert werden.

Erfindungsgemäß kann zusätzlich das vom Dampfabscheider 10 ausgehende Expanderrohr 11 durch den Vorkonditionierungstank 1 geführt sein und zur Abgabe von Kontakt-Abwärme an den Inhalt Vorkonditionierungstankes 1 ausgebildet sein.

Von Vorteil ist es dabei, wenn der Dampfabscheider bzw. der Zyklon 10 direkt über dem Vorkonditionierungstank 1 angeordnet ist und das im Wesentlichen senkrecht in den Vorkonditionierungstank 1 führende Expanderrohr 11 in etwa das Volumen einer Entnahmecharge aus dem Hydrolizer 8 aufweist. Über den Zeitraum eines Batch-Vorganges bleibt das abgelassene Heißmedium im Expanderrohr 11 und gibt Überschusswärme an das vom Rührwerk 2 über die Kontaktflächen des Expanderrohrs geführten Rohsubstrate ab.

Die dadurch erzielte Betriebstemperatur im Vorkonditionierungstank 1 beträgt etwa 70°C bis 90°C. Dadurch reduziert sich der Heizaufwand zum Erreichen des Betriebspunktes im Hydrolizer 8 (z.B. 180°C) beträchtlich.

Durch diese vorteilhafte Konstruktion entfallen sämtliche externe Wärmetauscher und Heiz/Kühlkreisläufe zur Vorwärmung der eingesetzten Rohsubstrate oder zur Abkühlung des hydrolisierten Substrates. Es kommt dadurch zu einer wesentlichen Vereinfachung des Systems und einer beträchtlichen Erhöhung der Energieeffizienz der Anlage.

Durch die erhöhte Temperatur im Vorkonditionierungstank 1 von ca. 70°C bis 90°C kann die Verweilzeit genutzt werden, um eine Vorhygienisierung des Substrates durchzuführen. Dadurch können bei Verarbeitung hygienisch bedenklicher Substrate die Mindestaufenthaltszeiten im Hydrolizer 8 weiter verkürzt werden, wodurch die Durchsatzleistung des Hydrolizers 8 erhöht wird.

Der Vorkonditionierungstank 1 dient weiters dazu, Substratmischungen aus fasrigen pflanzlichen Stoffen (Trockensubstanzgehalt ca. 30% bis 90%) vor der weiteren Behandlung durch Quellvorgänge Wasser zuzuführen. Dieser Vorgang wird durch die erhöhte Temperatur im Vorbehandlungsbehälter und die mechanischen Scherkräfte des Rührwerkes 2 begünstigt.

Aus dem Vorkonditionierungstank 1 wird das erhitzte und optimal angemaischte Substrat durch eine Verdrängerpumpe 4 zum Druckbehälter 5 der Beschickungseinrichtung 15 für den Hydrolizer 8 transportiert.

Der Druckbehälter 5 der Beschickungseinrichtung 15 kann mit einer Siebeinheit 6 ausgestattet sein, welche zur weiteren Entwässerung des aus dem Vorkonditionierungstank 1 zugeführten Substrates dient. Der Druckbehälter 5 weist neben einer ventilgesteuerten Beschickungsleitung 14 zum Hydrolizer 8 eine weitere ventilgesteuerte Verbindungsleitung 7 zur Herstellung eines Temperatur- und Druckausgleiches mit dem Hydrolizer 8 auf.

Der Druckbehälter 5 ist als sogenannte "Blow Gun" ausgeführt, d.h. der Behälter wird nach der Befüllung einer Charge nach außen druckdicht verschlossen, durch Druckausgleich mit dem Hydrolizer 8 über die ventilgesteuerte Verbindungsleitung 7 mit dem erhöhten Systemdruck beaufschlagt und - nach dessen (teilweiser) Entspannung - unter Ausnutzung des nun herrschenden Überdruckes in den Hydrolizer 8 überführt. Gleichzeitig mit dem Druckausgleich erfolgt auch eine Aufheizung des Rohsubstrates im Behälter 5 durch Angleichung an die Temperatur im Hydrolizer 8.

Durch die Integration einer Siebeinheit 6 im Druckbehälter 5 ist es möglich, den Behälterüberdruck zu nutzen, um überschüssiges, freies Wasser aus grobem Substrat (wie z.B. Stroh) zu entfernen. Dadurch reduziert sich der dem Hydrolizer zugeführte Massenstrom, so dass die zur Aufheizung erforderliche Energiemenge weiter reduziert werden kann.

Erfindungsgemäß kann ausgehend von der Siebeinheit 6 im Druckbehälter 5 eine zum Vorkonditionierungstank 1 führende Rezirkulationsleitung 16 für das in der Siebeinheit 6 abgepresste Flüssigsubstrat vorgesehen sein. Das Drainagewasser kann somit direkt oder nach Vermischung mit anderen Substraten dem weiteren Produktionsprozess zugeführt werden.

Erfindungsgemäß kann weiters die Rezirkulationsleitung 16 über einen im Expandertank 12 integrierten Wärmetauscher 13 geführt sein. Der Inhalt des Expandertankes 12 wird dadurch gekühlt, und gleichzeitig nimmt das durch den Wärmetauscher 13 geführte Rezyklat Wärmeenergie auf.

Nach der Befüllung des Hydrolizers 8 aus dem Druckbehälter 5 verläuft der Hydrolisierungsprozess über eine durchschnittliche Verweildauer von beispielsweise 30 Minuten bis zu mehreren Stunden. Im Anschluss an den Aufheizvorgang wird ein definiertes Volumen durch den Systemdruck ausgeschleust und durch die spontane Entspannung an der Druckblende 9 desintegriert.

Die Beschickung und Ausschleusung von Substrat in und aus dem Hydrolizer 8 erfolgt dabei in kurzer Zyklenfolge, z.B. 2 bis 4 Zyklen pro Stunde, und umfasst dabei nur einen Teil des Hydrolizer-Volumens, beispielsweise 10% bis 30%. Diese besondere Betriebsweise mit einer schnellen Abfolge von Beschickungs- und Entnahmezyklen für einen Teil des Reaktorvolumens wird in der Folge als quasikontinuierlich bezeichnet.

Die quasikontinuierliche Betriebsweise ergibt mehrere entscheidende Vorteile gegenüber bekannten kontinuierlichen und diskontinuierlichen Prozessen:
a) Durch den stoßweisen Ablass können große Blendenweiten mit hohem Durchsatz verwendet werden, wodurch Blendenverschleiß und Verstopfungen, wie sie typisch für kontinuierliche Verfahren sind, nicht auftreten.
b) Durch die Entnahme nur eines Teiles des Hydrolizer-Volumens wird das gesamte Substrat mit dem maximalen Entspannungseffekt, dem sogenannten "Schärfegrad", ausgeschleust, wodurch ein optimales Desintegrationsergebnis erzielt wird. Klassische diskontinuierliche Batchverfahren mit bei jedem Zyklus kompletter Reaktorentleerung haben eine unvermeidbaren Schlupf an gering desintegriertem Substrat, da mit fortschreitender Reaktorentleerung der für den Treibeffekt vorliegende Systemüberdruck kontinuierlich sinkt.
c) Klassische Batchverfahren weisen aufgrund der Betriebsweise einen zyklischen Bedarf an Beheizung auf, so dass eine erhöhte Spitzenleistung sowie ein diskontinuierlicher Verbrauch an Heizmedium zu bedienen ist. Durch die quasikontinuierliche Betriebsweise des Hydrolizers 8 kann die Beheizung permanent mit gleicher Leistung erfolgen, was z.B. der typischen Betriebsweise einer Biogasanlage entgegenkommt

Die Beheizung des Hydrolizers 8 erfolgt in beiden Ausführungsvarianten üblicherweise durch Dampf, Thermoöl oder einen Gasbrenner. Im Falle einer Kombination der Thermodruckhydrolyse mit einer Biogasanlage mit Co-Generation (Erzeugung von Elektrizität und Abwärme durch ein Blockheizkraftwerk BHKW oder eine gleichartige Verbrennungsmaschine) als typischer Anlagenkonfiguration kann eine Einrichtung 17 zur Zufuhr heißer Abgase der Co-Generation zur Direktbeheizung des Hydrolizers 8 verwendet werden.

Diese Betriebsweise führt zu einer weiteren energetischen Systemoptimierung.

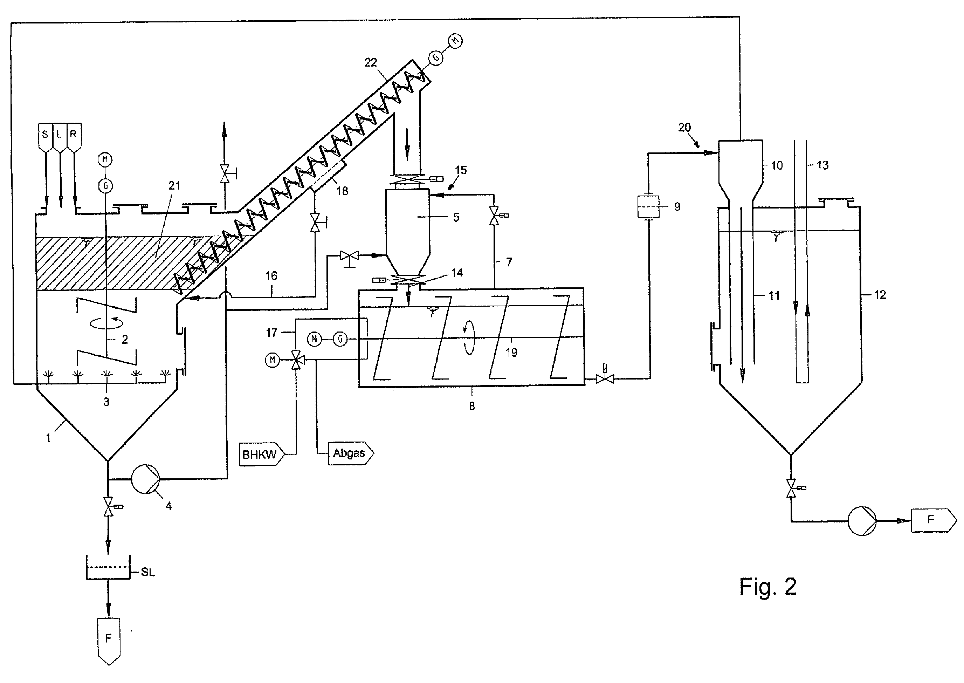

Das nach dem Austritt aus dem Hydrolizer 8 nahezu vollständig verflüssigte Substrat gelangt in den Zyklon 10, wobei der Gasanteil abgeschieden wird und der flüssige Anteil nach unten in das Expanderrohr 11 fließt, das einen für den Wärmeübergang optimierten geometrischen Querschnitt (rund, elliptisch oder vieleckig) aufweist. Der Zyklon 10 kann in kompakter Weise direkt oberhalb des Vorkonditionierungstankes 1 angeordnet sein, so dass das Ablaufrohr für das flüssige Substrat direkt durch den Vorkonditionierungstank 1 führt und zur Abgabe von Kontakt-Abwärme an den Inhalt des Vorkonditionierungstanks ausgebildet ist (siehe Fig. 1) oder direkt in den Expandertank 12 münden (siehe Fig. 2).

Das Expanderrohr 11 führt in der Variante gemäß Fig. 1 aus dem Vorkonditionierungstank 1 seitlich heraus in den Expandertank 12, aus welchem das Substrat beispielsweise einem Fermenter F einer Biogasanlage zugeführt wird.

Durch einen im Expandertank 12 integrierbaren Wärmetauscher 13 kann die hohe Systemtemperatur des Substrates (ca. 100°C) genutzt werden, um andere Medienströme wie z.B. nicht zu desintegrierendes Substrat (echte Flüssigkeiten) oder Brauchwasser zu erhitzen.

Die in Fig. 2 dargestellte Vorrichtung zur diskontinuierlichen Hydrolyse von organischen Substraten besteht im Wesentlichen aus folgenden Komponenten:
- einem mit Flüssigkeit gefüllten Vorkonditionierungstank 1 zur Aufnahme insbesondere fester, schwimmfähiger organischer Substrate mit einem Rührwerk 2 und einer Dampfverteilereinheit 3 (z.B. als spezieller Düsenstock zur Erzielung eines Flotationseffektes);
- einer Transportschnecke 22 zur Entnahme des organischen Substrates aus einem sich oberflächlich ausbildenden Schwimmkoffer 21, mit einer integrierten Siebeinheit 18 und einer Rezirkulationsleitung 16 für den Filtratrücklauf;
- einer Transferpumpe 4 zur Entnahme von (z.B. kontaminierter) Flüssigkeit aus dem Vorkonditionierungstank 1;
- einer Beschickungseinrichtung 15 mit einem Druckbehälter 5 (Blow Gun) einer Beschickungsleitung bzw. Schleuse 14 und einer ventilgesteuerten Verbindungsleitung 7 zum Hydrolizer 8;
- einem Hydrolizer 8 mit einem Rührwerk 19 zur Durchführung der Thermodruckhydrolyse;
- einer ventilgesteuerten Entspannungseinrichtung bestehend aus einer Druckblende 9, einem Dampfabscheider 10 (z.B. Zyklon) und einem Expanderrohr 11, welches in einen Expandertank 12 führt;
- einer Einrichtung 17 zur Beheizung des Hydrolizers (z.B. mit Abgas);
- einem Expandertank 12 mit einem integrierten Wärmetauscher 13.

Die Variante gemäß Fig. 2 eignet sich insbesondere für agrarische Abfälle wie Stroh, Mist, oder welkes Heu, sowie andere stark verholzte Materialien wie Schilf oder Laub, deren Eigenschaften bisher eine Verwertung z.B. in Biogasanlagen erschwert bzw. vereitelt haben.

Die Neigung zum Aufschwimmen und Kompaktieren dieser sehr fasrigen Stoffe sowie eine nur sehr langsame Hydrolyse und damit verzögerte Umsetzung der Substrate sind die hier wesentlichen Substrateigenschaften.

Zu den definierten Zeitpunkten, in welchen Heißdampf aus dem Dampfabscheider 10 zugeführt wird, befindet sich das Rührwerk 2 im Vorkonditionierungstank 1 im Ruhezustand bzw. in sehr langsamer Rotation, so dass fasrige pflanzliche Substrate aufschwimmen. Der eintretende Heißdampf verstärkt diesen Effekt, indem sich die Dampfblasen an die Festteilchen haften, und diese in einem Flotationseffekt an der Reaktoroberfläche verdichten und einen Schwimmkoffer 21 bilden.

Neben dem Effekt der Substratsammlung bewirkt der verdichtete Substrat-Polster auch einen verbesserten Wärmeübergang vom Dampf auf das Substrat. Dieser Vorgang wird im Weiteren als Dampfflotation bezeichnet.

Aus dem Vorkonditionierungstank 1 wird das erhitzte und optimal angemaischte Substrat auf zwei möglichen Wegen zum Druckbehälter 5 der Beschickungseinrichtung 15 transportiert:

### a) Schwimmfähige Substrate

Fasrige, mit Hilfe der Dampfflotation aufschwimmende Substrate, wie z.B. aus Pflanzenabfällen und Energiepflanzen, werden durch eine Transportschnecke 22 aus dem kompakten Schwimmkoffer 21 abgezogen, wobei durch langsame Rotation des Rührwerkes 2 das Heranführen des Substrates an die Transportschnecke 22 verbessert werden kann. Durch den Einsatz einer Siebeinrichtung 18 im Fördertrog der Schnecke 22 kann eine Rückführung von überschüssigem Prozesswassers im gewünschten Ausmaß erfolgen. Dadurch kann eine Reduktion der Menge des im Hydrolizer 8 zu behandelnden Materials erzielt werden. Die abgeschiedene Flüssigkeit läuft in den Vorkonditionierungstank 1 zurück.

### b) Flüssige oder sinkende Substrate

Nicht aufschwimmende Substrate werden über den trichterförmigen Bodenauslass des Vorkonditionierungsbehälters 1 durch eine geeignete Pumpe 4 zum Druckbehälter 5 der Beschickungseinrichtung 15 gefördert.

Ebenso wird diese Pumpe 4 verwendet, um zyklisch aufkonzentriertes (insbesondere organische Säuren enthaltendes) und damit korrosives Anmaischwasser aus dem Vorkonditionierungstank 1 abzuschlagen.

## Patentansprüche

1. Vorrichtung zur diskontinuierlichen Hydrolyse von organischen Substraten, insbesondere von Energiepflanzen, sowie pflanzlichen und tierischen Abfällen, mit einem Vorkonditionierungstank (1) zur Aufnahme fester und/oder flüssiger organischer Substanzen, mit einer Beschickungseinrichtung (15) zur Befüllung eines Hydrolizers (8) mit den Substraten aus dem Vorkonditionierungstank (1), sowie mit einer Entspannungseinrichtung (20), die zur Druckentspannung der Substrate nach der Behandlung im Hydrolizer (8) einen Expandertank (12) aufweist, **dadurch gekennzeichnet, dass** die Entspannungseinrichtung (20) einen Dampfabscheider (10) aufweist, der dem Expandertank (12) vorgeschaltet und stromabwärts einer Druckblende (9) angeordnet ist, wobei eine Einrichtung zur direkten Beaufschlagung der zugeführten organischen Substrate mit aus dem Dampfabscheider (10) abgeschiedenem Heißdampf vorgesehen ist.

2. Vorrichtung nach Anspruch 1, **dadurch gekennzeichnet, dass** der Dampfabscheider als Zyklon (10) ausgebildet ist und eine Dampfverteilereinheit (3), beispielsweise eine Düsenstockeinheit, speist, die direkt im Vorkonditionierungstank (1) für die organischen Substrate angeordnet ist.

3. Vorrichtung nach Anspruch 1 oder 2, **dadurch gekennzeichnet, dass** ein vom Dampfabscheider (10) ausgehendes Expanderrohr (11) durch den Vorkonditionierungstank (1) geführt ist und zur Abgabe von Kontakt-Abwärme an den Inhalt des Vorkonditionierungstanks (1) ausgebildet ist.

4. Vorrichtung nach Anspruch 3, **dadurch gekennzeichnet, dass** der Dampfabscheider (10) direkt über dem Vorkonditionierungstank (1) angeordnet ist und das im Wesentlichen senkrecht in den Vorkonditionierungstank (1) führende Expanderrohr (11) in etwa das Volumen einer Entnahmecharge aus dem Hydrolizer (8) aufweist.

5. Vorrichtung nach Anspruch 1 oder 2, **dadurch gekennzeichnet, dass** ein vom Dampfabscheider (10) ausgehendes Expanderrohr (11) direkt in den Expandertank (12) mündet.

6. Vorrichtung nach einem der Ansprüche 1 bis 5, **dadurch gekennzeichnet, dass** im Vorkonditionierungstank (1) ein Rührwerk (2) angeordnet ist.

7. Vorrichtung nach einem der Ansprüche 1 bis 6, **dadurch gekennzeichnet, dass** die Beschickungseinrichtung (15) zur Befüllung des Hydrolizers (8) einen Druckbehälter (5) aufweist, der neben einer ventilgesteuerten Beschickungsleitung (14) zum Hydrolizer (8) eine weitere ventilgesteuerte Verbindungsleitung (7) zur Herstellung eines Temperatur- und Druckausgleichs mit dem Hydrolizer (8) aufweist.

8. Vorrichtung nach Anspruch 7, **dadurch gekennzeichnet, dass** der Druckbehälter (5) der Beschickungseinrichtung (15) mit einer Siebeinheit (6) ausgestattet ist, welche zur Entwässerung des aus dem Vorkonditionierungstank (1) zugeführten Substrates dient.

9. Vorrichtung nach Anspruch 8, **dadurch gekennzeichnet, dass** ausgehend von der Siebeinheit (6) des Druckbehälters (5) eine zum Vorkonditionierungstank (1) führende Rezirkulationsleitung (16) für das in der Siebeinheit (6) abgepresste Flüssigsubstrat vorgesehen ist.

10. Vorrichtung nach Anspruch 9, **dadurch gekennzeichnet, dass** die Rezirkulationsleitung (16) über einen im Expandertank (12) integrierten Wärmetauscher (13) geführt ist.

11. Vorrichtung nach Anspruch 7, **dadurch gekennzeichnet, dass** die Beschickungseinrichtung (15) eine in den Vorkonditionierungstank (1) ragende Transportschnecke (22) zur Förderung eines oberflächlichen Schwimmkoffers (21) aufweist, der aus festen, faserigen Bestandteilen der vorzugsweise pflanzlichen Abfälle besteht.

12. Vorrichtung nach Anspruch 11, **dadurch gekennzeichnet, dass** die Transportschnecke (22) eine Siebeinheit (18) aufweist, welche zur Vorentwässerung des geförderten Substrats dient.

13. Vorrichtung nach einem der Ansprüche 1 bis 12, **dadurch gekennzeichnet, dass** die Vorrichtung Teil einer Biogasanlage mit einem Blockheizkraftwerk ist, wobei eine Einrichtung (17) zur Zufuhr heißer Abgase des Blockheizkraftwerks zur Beheizung des Hydrolizers (8) vorgesehen ist.

14. Verfahren zur diskontinuierlichen Hydrolyse von organischen Substraten, insbesondere von Energiepflanzen, sowie pflanzlichen und tierischen Abfällen, mittels Thermodruckhydrolyse (Steam-Explosion) **dadurch gekennzeichnet, dass** aus den in der Thermodruckhydrolyse behandelten Substraten unmittelbar nach dem Ausschleusen einer Teilmenge des Substrats durch Druckentspannung Heißdampf abgeschieden wird und zur Erhitzung der dem Hydrolyseverfahren zugeführten organischen Substrate eingesetzt wird, wobei der Heißdampf direkt in das angemaischte organische Substrat eingeblasen wird.

15. Verfahren nach Anspruch 14, **dadurch gekennzeichnet, dass** der zugeführte Heißdampf zur Abtrennung fester, faseriger Bestandteile der vorzugsweise pflanzlichen Abfälle und zur Bildung eines oberflächlichen Schwimmkoffers eingesetzt wird.

16. Verfahren nach Anspruch 14 oder 15, **dadurch gekennzeichnet, dass** die der Thermodruckhydrolyse zugeführten angemaischten, organischen Substrate zuvor entwässert werden, so dass nur noch geringere Substratmengen auf das für die Thermodruckhydrolyse erforderliche Temperaturniveau gebracht werden müssen.

## Claims

1. Apparatus for discontinuous hydrolysis of organic substrates, in particular energy crops, as well as vegetable or animal waste, which comprises a pre-conditioning tank (1) for receiving solid and/or liquid organic substances, and a charging device (15) for filling a hydrolizer (8) with the substrates from said pre-conditioning tank (1), and further an expansion device (20), which has a flash tank (12) for depressurizing the substrates after treatment in said hydrolizer (8), **characterised in that** the expansion device (20) is provided with a steam separator (10) preceding the flash tank (12) and placed downstream of a pressure baffle (9), a device for directly subjecting the input of organic substrates to superheated steam from said steam separator (10) being furnished.

2. Apparatus according to claim 1, **characterised in that** the steam separator is configured as a cyclone separator (10) and feeds a steam distribution unit (3), for instance a tuyere stock, which is directly located in the pre-conditioning tank (1) for the organic substrates.

3. Apparatus according to claim 1 or 2, **characterised in that** an expansion pipe (11) departing from the steam separator (10) passes through the pre-conditioning tank (1) and is designed to transfer contact waste heat to the contents of said pre-conditioning tank (1).

4. Apparatus according to claim 3, **characterised in that** the steam separator (10) is placed directly above the pre-conditioning tank (1) and that the expansion pipe (11) extending essentially vertically into the pre-conditioning tank (1) has roughly the volume of one exit batch from the hydrolizer (8).

5. Apparatus according to claim 1 or 2, **characterised in that** an expansion pipe (11) departing from the steam separator (10) directly opens into the flash tank (12).

6. Apparatus according to any of claims 1 to 5, **characterised in that** an agitator (2) is provided in the pre-conditioning tank (1).

7. Apparatus according to any of claims 1 to 6, **characterised in that** the charging device (15) for filling the hydrolizer (8) has a pressure bin (5), which is provided with a valve-controlled charging line (14) to the hydrolizer (8) and an additional valve-controlled connecting line (7) for establishing temperature and pressure equilibrium with the hydrolizer (8).

8. Apparatus according to claim 7, **characterised in that** the pressure bin (5) of the charging device (15) is furnished with a sieve unit (6) for removing water from the substrate fed from the pre-conditioning tank (1).

9. Apparatus according to claim 8, **characterised in that** departing from the sieve unit (6) of the pressure bin (5) a recirculation line (16) to the pre-conditioning tank (1) is provided for the liquid substrate obtained in the sieve unit (6).

10. Apparatus according to claim 9, **characterised in that** the recirculation line (16) passes through a heat exchanger (13) integrated in the flash tank (12).

11. Apparatus according to claim 7, **characterised in that** the charging device (15) is provided with a screw conveyor (22) extending into the pre-conditioning tank (1) for transporting a surface mat (21) of solid, fibrous components of the preferably vegetable waste floating on the surface of the tank.

12. Apparatus according to claim 11, **characterised in that** the screw conveyor (22) has a sieve unit (18) for pre-extracting water from the substrate transported.

13. Apparatus according to any of claims 1 to 12, **characterised in that** the apparatus is part of a biogas plant with a combined heat and power unit, where a means (17) is provided for supplying hot exhaust gas from the combined heat and power unit for heating the hydrolizer (8).

14. Method for discontinuous hydrolysis of organic substrates, in particular energy crops, as well as vegetable and animal waste, by means of thermal pressure hydrolysis (steam explosion), **characterised in that** from the substrates treated by thermal pressure hydrolysis superheated steam is separated by flashing immediately after exit of part of the substrate from the hydrolizer and is used for heating the organic substrates fed to the hydrolysis process, the superheated steam being blown directly into the mashed organic substrate.

15. Method according to claim 14, **characterised in that** the introduced superheated steam is used for separating solid, fibrous components of the preferably vegetable waste in such a way that they form a floating surface mat.

16. Method according to claim 14 or 15, **characterised in that** water is removed from the mashed organic substrates prior to feeding them into the process of thermal pressure hydrolysis, such that smaller amounts of substrate need to be heated to the temperature required by thermal pressure hydrolysis.

## Revendications

1. Dispositif d'hydrolyse discontinue de substrats organiques en particulier de plantes énergétiques ainsi que de déchets végétaux et animaux, comportant une cuve de conditionnement préalable (1) pour la réception de substances organiques solides et/ou liquides, un dispositif de chargement (15) pour remplir un hydrolyseur (8) avec les substrats provenant de la cuve de conditionnement préalable (1), ainsi qu'un dispositif de détente (20) qui comporte une cuve de détente (12), pour détendre la pression du substrat après le traitement dans l'hydrolyseur (8),
**caractérisé en ce que**
le dispositif de détente (20) comporte un séparateur de vapeur (10) qui est monté en amont de la cuve détente (12) et en aval d'un diaphragme de pression (9), un dispositif pour la fourniture directe au substrat organique transféré de vapeur chaude séparée du séparateur de pression (10) étant prévu.

2. Dispositif conforme à la revendication 1,
**caractérisé en ce que**
le séparateur de vapeur est réalisé sous la forme d'un cyclone (10) et alimente une unité de répartition de vapeur (3), par exemple une unité de canne à buses, qui est disposée directement dans la cuve de conditionnement préalable (1) pour les substrats organiques.

3. Dispositif conforme à la revendication 1 ou 2,
**caractérisé en ce qu'**
un tube de détente (11) partant du séparateur de vapeur (10) passe au travers de la cuve de conditionnement préalable (1) et est réalisé pour dégager de la chaleur dissipée par contact à la partie interne de la cuve de conditionnement préalable (1).

4. Dispositif conforme à la revendication 3,
**caractérisé en ce que**
le séparateur de pression (10) est monté directement au-dessus de la cuve de conditionnement préalable (1) et le tube de détente (11) qui s'étend essentiellement verticalement dans la cuve de conditionnement préalable (1), présente essentiellement le volume d'une charge de soutirage provenant de l'hydrolyseur (8).

5. Dispositif conforme à la revendication 1 ou 2,
**caractérisé en ce qu'**
un tube de détente (11) sortant du séparateur de vapeur (10) débouche directement dans la cuve de détente (12).

6. Dispositif conforme à l'une des revendications 1 à 5,
**caractérisé en ce qu'**
un dispositif agitateur (2) est monté dans la cuve de conditionnement préalable (1).

7. Dispositif conforme à l'une des revendications 1 à 6,
**caractérisé en ce que**
le dispositif de chargement (15) comporte, pour remplir l'hydrolyseur (8), un récipient sous pression (5) qui est équipé, outre d'une conduite d'alimentation (14) vers l'hydrolyseur (8) commandée par une soupape, d'une autre conduite de liaison (7) commandée par une soupape pour permettre un équilibrage de la température et de la pression avec l'hydrolyseur (8).

8. Dispositif conforme à la revendication 7,
**caractérisé en ce que**
le récipient sous pression (5) du dispositif de chargement (15) est équipé d'une unité de criblage (6) qui a pour fonction de déshydrater le substrat sortant de la cuve de conditionnement préalable (1).

9. Dispositif conforme à la revendication 8,
**caractérisé en ce que**
de l'unité de criblage (6) du récipient sous pression (5), part une conduite de recirculation (16) du substrat liquide extrait par pression dans cette unité de criblage (6), conduisant à la cuve de conditionnement préalable (1).

10. Dispositif conforme à la revendication 9,
**caractérisé en ce que**
la conduite de recirculation (16) passe dans un échangeur de chaleur (13) intégré dans la cuve de détente (12).

11. Dispositif conforme à la revendication 7,
**caractérisé en ce que**
le dispositif de chargement (15) comporte une vis transporteuse (22) qui pénètre dans la cuve de conditionnement préalable (1) pour transporter un tampon flottant superficiel (21) constitué d'éléments solides fibreux des déchets de préférence végétaux.

12. Dispositif conforme à la revendication 11,
**caractérisé en ce que**
la vis transporteuse (22) comporte une unité de criblage (18) qui sert à effectuer une déshydratation préalable du substrat alimenté.

13. Dispositif conforme à l'une des revendications 1 à 12,
**caractérisé en ce que**
le dispositif constitue une partie d'une installation de biogaz comportant une centrale thermique de bloc, un dispositif (17) pour transférer les gaz d'échappement chauds de la centrale thermique par bloc étant prévu pour chauffer l'hydrolyseur (8).

14. Procédé d'hydrolyse discontinue de substrats organiques en particulier de plantes énergétiques ainsi que de déchets végétaux ou animaux par hydrolyse thermique sous pression (explosion à la vapeur),
**caractérisé en ce qu'**
on sépare de la vapeur chaude des substrats traités par hydrolyse thermique sous pression, immédiatement après la séparation par éclusage d'une quantité partielle du substrat, par détente de pression, et on la met en oeuvre pour chauffer les substrats organiques alimentant le procédé d'hydrolyse, la vapeur chaude étant directement soufflée dans le substrat organique mis à tremper.

15. Procédé conforme à la revendication 14,
**caractérisé en ce que**
la vapeur chaude transférée est mise en oeuvre pour séparer des composants solides fibreux des déchets, de préférence végétaux et pour former un tampon flottant superficiel.

16. Procédé conforme à la revendication 14 ou 15,
**caractérisé en ce que**
les substrats organiques mis à tremper alimentant l'hydrolyse thermique sous pression, sont d'abord déshumidifiés de sorte que seules de faibles quantités de substrat doivent être amenées au niveau de température nécessaire pour l'hydrolyse thermique sous pression.
